# EUROPÄISCHE PATENTANMELDUNG

(11) **EP 0 745 460 A1**
(43) Veröffentlichungstag der Anmeldung: **04.12.1996**
(21) Anmeldenummer: 95108504.2
(22) Anmeldetag: 02.06.1995
(51) Int. Cl.: B25D 17/08

(54) **Werkzeugaufnahme für Schlagmaschinen**

(71) Anmelder: Grünig & Elmiger AG Jagd- und Sportwaffenfabrik, CH-6102 Malters (CH)
(72) Erfinder: Heinz, Elmiger, CH-6102 Malters (CH)
(74) Vertreter: Kemény AG Patentanwaltbüro

(57) **Zusammenfassung**

Der Adapter (3) für die Aufnahme und Verbindung von Werkzeugen (2) mit einer Schlagmaschine (1) weist radial zur Adapterachse verschiebbare Haltekeile (10,11) auf, welche in eine entsprechende Nut (12) der Befestingungsachse (13) des Werkzeuges (2) eingreifen können. Die radiale Bewegung der Haltekeile (10,11) wird durch Verdrehen eines Hohlkörpers (5) gegenüber einem Mitnehmerteil (6) resp. eines Aufnahmeelementes (14) erreicht. Für die Fixierung des Verdrehwinkels der Teile untereinander sind Rastnocken (8;15) vorgesehen. Diese werden vorteilhafterweise durch ringförmige Federelemente (9) aus Kunststoff betätigt. Ein solcher erfindungsgemässer Adapter eignet sich insbesondere für die Anwendung im Medizinalsektor, da an seiner Aussenseite keine beweglichen oder verschraubten Kleinteile vorhanden sind.

## Beschreibung

Die vorliegende Erfindung betrifft einen Adapter gemäss Oberbegriff von Anspruch 1.

Eine Schlagmaschine weist als Arbeitsmittel einen translatorisch bewegten Stössel auf, welcher ein Werkzeug hin- und herbewegt. Für die Aufnahme und Befestigung von Werkzeugen in Schlagmaschinen werden herkömmlicherweise Adapter verwendet. Diese dienen der Koppelung des Werkzeuges mit dem beweglichen Stössel der Maschine. Dabei wird in der Regel der Adapter fest mit dem Stössel verbunden, beispielsweise mittels einer Verschraubung, oder er wird direkt in das Ende des Stössels integriert. Andererseits soll das Werkzeug möglichst einfach mit der Maschine verbunden und wieder gelöst werden können, wobei im verbundenen Zustand eine möglichst spielfreie Verbindung mit dem Stössel erfolgen sollte. Im Weiteren unterliegt der Adapter einen hohen Arbeitsbelastung, er muss demgemäss eine hohe mechanische Festigkeit aufweisen.

Hier stellt sich nun insbesondere im Medizinalsektor weiter das Problem, einen Adapter zu verwenden, welcher beim Einsatz der Maschine in keinem Fall einen Fremdkörper verlieren kann oder sonstwie unkontrolliert Fremdkörper, wie beispielsweise Schmiermittel, abgeben darf. Weiter müssen solche Geräte nach jedem Einsatz sterilisiert werden können.

Die Aufgabe der vorliegenden Erfindung bestand nun darin, einen solchen Adapter zu finden, bei welchem die Bedienung, d.h. das Befestigen resp. Lösen des Werkzeuges, möglichst einfach erfolgen kann und der gleichzeitig derart aufgabaut ist, dass sich im Betrieb keine Teile vom Adapter lösen oder davon abfallen können.

Diese Aufgabe wird erfindungsgemäss entsprechend dem Kennzeichen von Anspruchs 1 gelöst.

Durch die erfindungsgemäss Anordnung der Haltekeile, welche gegen eine im Adapterquerschnitt ovalförmige Nut im Hohlkörper federnd anliegen, wird eine zuverlässige Befestigung des Werkzeuges durch blosses Verdrehen des Hohlkörpers bezüglich dem Mitnehmerteil erreicht. Indem die Haltekeile, d.h. die beweglichen Kleinteile, vollständig im Innern des Adapters angeordnet sind, wird auch ein ungewolltes Loslösen dieser Teile im Betrieb vermieden.

Weitere bevorzugte Ausführungsformen ergeben sich aus den Ansprüchen 2 bis 6.

Insbesondere reduziert die Verwendung von elastischen Ringen die Bruchgefahr und damit die Gefahr der Erzeugung von unerwünschten losen Fremkörpern im Betrieb des Gerätes, wie sie bei der Verwendung von metallischen Federn auftreten kann.

Ein weiterer Vorteil besteht in der Möglichkeit, den eigentlichen Werkzeughalter gegenüber dem Mitnehmerteil vorzugweise in diskreten Abständen zu verdrehen. Dies dient insbesondere der Bedienungserleichterung.

Ein solcher Adapter eignet sich insbeondere vorteilhaft für den Einsatz im Medizinalsektor. Durch die sehr kompakte Bauweise lässt sich der Adapter überdies einfach sterilisieren, wobei die Funktionstüchtigkeit auch bei sehr raschem Einsatz unmittelbar nach einer Sterilisation erhalten bleibt.

Ein Ausführungsbeispiel der Erfindung wird nachstehend anhand von Zeichnungen noch näher erläutert. Es zeigen
Fig. 1 schematisch die Ansicht einer Schlagmaschine mit eingesetztem Werkzeug (Raspel);
Fig. 2 den Längsschnitt eines erfindungsgemässen Adapters mit eingesetztem Werkzeug;
Fig. 3 einen Querschnitt durch den Adapter von Fig.2; und
Fig. 4 einen weiteren Querschnitt durch den Adapter von Fig. 2.

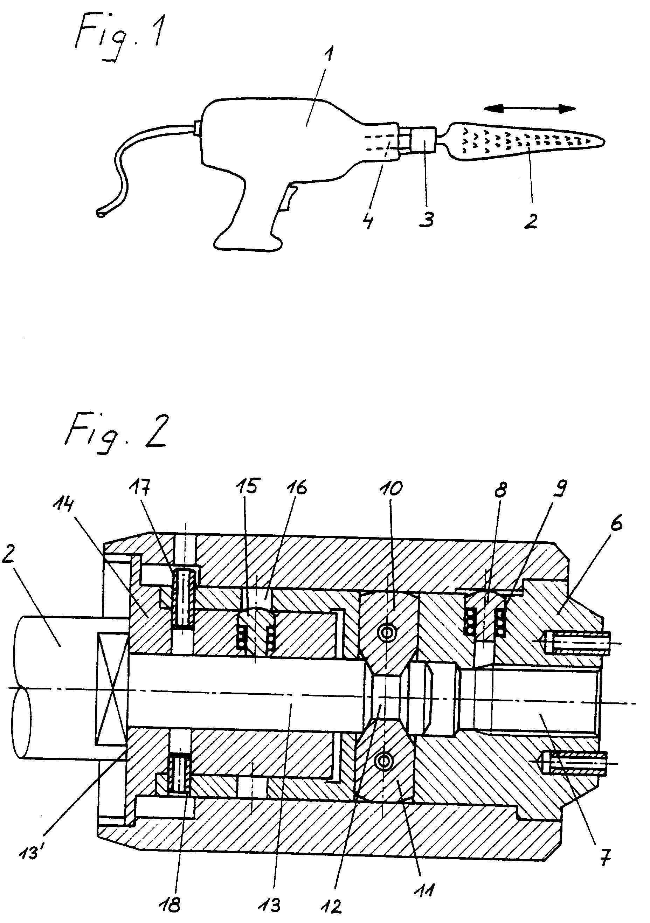

In Figur 1 ist schematisch eine Schlagmaschine 1 mit einem eingesetzten Werkzeug 2, hier beispielsweise einer Raspel, dargestellt. Das Werkzeug 2 wird mittels eines Adapters 3 mit dem Stössel 4 der Schlagmaschine 1 verbunden. Unter einer Schlagmaschine wird hier eine Maschine mit einem translatorisch vor- und rückwärtsbewegten Stössel verstanden. Solche Maschinen können beispielweise elektrisch oder mittels Druckluft betrieben sein. Der Adapter 3 im hier dargestellten Fall zur Betätigung einer Raspel 2 ist hohen mechanischen Belastungen ausgesetzt und muss gleichwohl das Werkzeug 2 spielfrei und verdrehsicher halten.

Gerade für den Einsatz in kritischen Bereichen, wie beispielsweise in der Medizin, werden weitere Anforderungen an solche Maschinen und damit auch an den Adapter gestellt. So dürfen sich im Betrieb keine Teile oder Stoffe vom Werkzeug oder der Maschine lösen, wie beispielsweise Betriebs- oder Schmierstoffe oder im Betrieb abgebrochene Teile des Adapters.

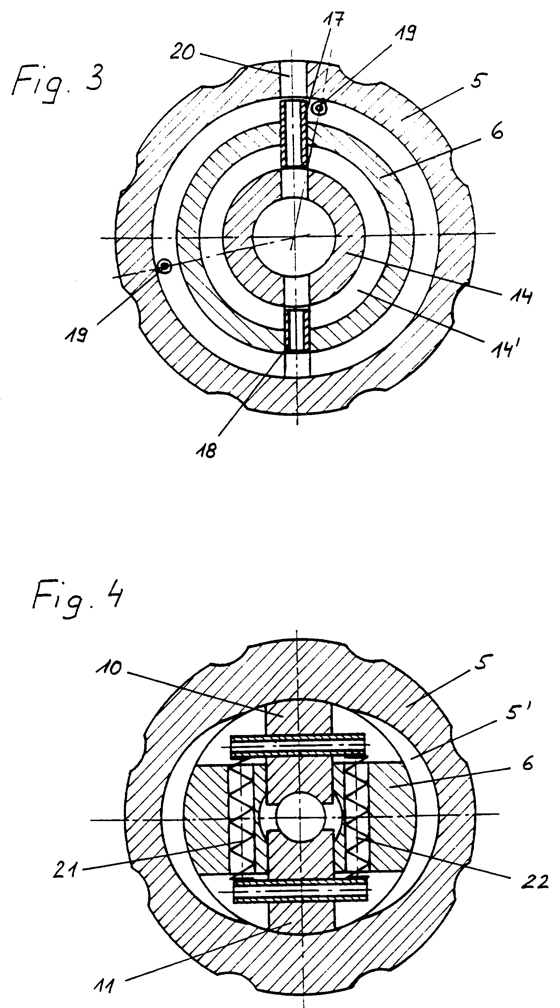

In Figur 2 ist nun der Längsschnitt durch einen erfindungsgemässen Adapter dargestellt, welche insbesondere auch diese zusätzlichen Anforderungen erfüllt. Der Adapter ist mit eingesetztem Werkzeug 2 dargestellt. Im zylindrischen Hohlmantel 5 sind einerseits der Mitnehmer 6 angeordnet, welcher beispielsweise mittels eines Gewindeloches 7 mit dem Stössel der Maschine (hier nicht dargestellt) verbunden werden kann. Der Mitnehmer 6 ist verdrehbar im Hohlmantel 5 gelagert. Mittels eines Rastnockens 8 kann der Hohlmantel 5 gegenüber dem Mitnehmer 6 in einer definierten Position fixiert werden. Vorzugsweise wird das Federelement 9 des Rastnockens 8 aus einem oder mehreren Kunststoffringen, beispielsweise Gummiringen, gebildet. Im Gegensatz zur Verwendung von Metallfedern besteht damit keine Gefahr, dass bei einem Bruch des Federelementes 9 Bruchstücke als Fremdkörper aus dem Adapterinnern herausfallen können.

Im Mitnehmer 6 sind hier beispielsweise zwei einander gegenüberliegende Festhaltekeile 10 und 11 radial verschiebbar gelagert. Diese greifen in der in Figur 1 gezeigten Lage in die entprechend keilförmig geformte Nut 12 des Werkzeugschaftes 13 ein und fixieren damit das Werkzeug 2 fest im Adapter 3.

Weiter ist ein Werkzeughalter 14 vorgesehen, welcher ebenfalls verdrehbar im Mitnehmer 6 gelagert ist. Er dient zur Aufnahme des Werkzeugschaftes 13, resp. dem Anliegen der Werkzeugschaftschulter 13'. An diesem Werkzeughalter 14 ist ebenfalls ein Rastnocken 15 vorgesehen, welcher in mehrere, an der Innenfläche des Mitnehmers 6 angebrachte Rastöffnungen 16 eingreifen kann, um die Lage des Werkzeughalters 14 gegenüber dem Mitnehmer 6 zu fixieren. Der Rastnocken 15 ist derart in einer Bohrung des Werkzeughalters 14 angeordnet, dass er bei eingeführtem Werkzeug 2, d.h. bei eingeführtem Werkzeugschaft 13, in eine Rastöffnung 16 eingreifen muss. Damit wird erreicht, dass der Werkzeughalter 14 nur dann gegenüber dem Mitnehmer 6 verdreht werden kann, solange das Werkzeug 2, resp. der Werkzeugschaft 13, nicht vollständig in den Werkzeughalter 14 eingeführt worden ist. Sobald das Werkzeug 2 vollständig eingeführt ist, kann der Werkzeughalter 14 und damit auch das Werkzeug 2 selbst nicht mehr gegenüber dem Mitnehmer 6 verdreht werden.

Der Werkzeughalter 14 ist mittels Federzapfen 17 und 18 mit dem Mitnehmer 6 verbunden. Diese sind derart angeordnet, dass eine axiale Verschiebung dieser beiden Teile nicht möglich ist, aber ein gegenseitiges Verdrehen ermöglicht ist. In Figur 3 ist der Querschnitt durch den Adapter im Bereich der beiden Federzapfen 17,18 dargestellt. Die beiden Zapfen 17,18 sitzen fest in Bohrungen des Mithmehrs 6, wobei ihre Enden in eine hier beispielsweise vollständig umlaufende Nut 14' des Werkzeughalters 14 reichen. Diese Anordnung hat nun den Vorteil, dass keines dieser Befestigungsteile 17,18 bei einem ungewollten Loslösen aus der Bohrung als Fremdkörper nach Aussen gelangen kann. Die einzige Möglichkeit, diese Verbindung wieder zu lösen besteht darin, die Federzapfen 17 und 18 jeweils entweder von Innen durch die Bohrung 20 im Hohlmantel 5 nach Aussen zu schlagen oder umgekehrt von Aussen bei nichteingeführtem Werkzeug 2 nach Innen zu schlagen. Hierfür sind allerdings Werkzeuge nötig; von selbst können die Federzapfen 17 und 18 das Gehäuseinnere nicht verlassen.

Um eine Begrenzung des Verdrehwinkels zwischen dem Mitnehmer 6 und dem Hohlmantel 5 zu erreichen, können, wie in Figur 3 dargestellt, Anschlagbolzen 19 vorgesehen sein. Dabei können einer oder beide Federzapfen 17,18 beim Verdrehen an den resp. die Anschlagbolzen 19 zum Anschlag gelangen.

Die Wirkungsweise der Festhaltekeile 10 und 11 ist aus dem in Figur 4 dargestellten Querschnitt durch den Adapter in diesem Bereich ersichtlich. Die Festhaltekeile 10 und 11 sind an Führungsflächen des Mitnehmers 6 radial beweglich geführt. Sie werden durch zwei Federelemente 21 und 22 gegen Aussen an die Wandung des Hohlmantels 5 gedrückt. Diese Federelemente 21,22 können beispielsweise, wie in Figur 4 dargestellt, Druckfedern sein. Bei einem Verdrehen des Mitnehmers 6 gegenüber dem Hohlmantel 5 folgen die Festhaltekeile 10 und 11 der Kontur der Hohlmantelwandung an dieser Stelle. Diese ist erfindungsgemäss als ovale Nut ausgebildet. Wenn nun aus der in Figur 4 dargestellten verriegelten Position der Mitnehmer 6 beispielsweise ca. 90° gegenüber dem Hohlmantel 5 verdreht wird, so werden die beiden Festhaltekeile 10 und 11 radial gegen Aussen verschoben und geben damit den Werkzeugschaft 13 frei. Damit kann das Werkzeug 2 aus dem Adapter herausgezogen werden.

Der hier vorgestellte Adapter zeichnet sich einerseits durch seine einfache Bedienung aus, indem der Schaft 13 des Werkzeuges 2 einfach in den Adapter eingeführt zu werden braucht, um danach beispielsweise durch eine Vierteldrehung am Hohlmantel 5 verriegelt zu werden. Andererseits weist der Adapter an der Aussenseite keine Befestigungsteile auf, welche sich von selbst lösen könnten. Weiter ist zusätzlich eine Verdrehmöglichkeit des Werkzeuges 2 vorgesehen, welche je nach Einsatzart den Bedienungskomfort wesentlich erhöhen kann.

Es ist selbstverständlich, dass sich ein solcher Adapter für alle Maschinen eignet, bei welchen achsiale Stoss- und Ziehbewegungen auftreten. Insbesondere eignet sich ein solcher Adapter aber hervorragend für den Medizinalbereich, gerade wegen des kompakten und einfachen Aufbaues.

## Patentansprüche

1. Adapter für Schlagmaschinen (1), welcher Mittel zur Verbindung mit einem Arbeitsstössel (4) der Maschine und Mittel zum Befestigen und Lösen von Werkzeugen (2) mit einer zylindrischen Befestigungsachse (13) mit umlaufender Nut (12) aufweist, dadurch gekennzeichnet, dass ein zylindrischer Hohlkörper (5) vorgesehen ist, welcher verdrehbar einen zylindrischen Mitnehmerteil (6) für die Verbindung mit dem Arbeitsstössel, mindestens zwei radial zur Hohlkörperachse bewegliche Haltekeile (10,11) im Innern des Hohlkörpers (5) zum Eingreifen in die Nut (12) des Werkzeuges (2) sowie eine bezüglich des Hohlkörpers (5) verdrehbares, zylindrisches Aufnahmeelement (14) für die Befestigungsachse (13) des Werkzeuges (2) aufweist, wobei die Haltekeile (10,11) gegeneinander federnd verspannt sind und radial gegen eine Führungsnut (5') der Innenwandung des Hohlkörpers (5) anliegen, welche bezüglich der Hohlkörperachse einen ovalen Querschnitt aufweist.

2. Adapter nach Anspruch 1, dadurch gekennzeichnet, dass der Verdrehwinkel des Aufnahmeelementes (14) in diskreten Schritten, mittels eines Rastnockens (15), bezüglich dem Mitnehmerteil (6) eingestellt werden kann.

3. Adapter nach Anspruch 2, dadurch gekennzeichnet, dass der Rastnocken (15) derart in einer Bohrung des Aufnahmelelementes (14) angeordnet ist, dass in nichteingerastetem Zustand ein Ende des Rastnockens in die Öffnung für die Aufnahme der Befestigungsachse (13) des Werkzeuges (2) hineinragt.

4. Adapter nach einem der Ansprüche 1 bis 3, dadurch gekennzeichnet, dass Mittel (17,18,19) für die Beschränkung des Verdrehwinkels zwischen dem Hohlkörper (5) und dem Mitnehmerteil (6) vorgesehen sind.

5. Adapter nach einem der Ansprüche 2 bis 4, dadurch gekennzeichnet, dass die Federlemente (9) der Rastnocken (8;15) aus Ringen aus elastischem Kunststoff bestehen.

6. Adapter nach einem der Ansprüche 1 bis 5, dadurch gekennzeichnet, dass das Mitnehmerteil (6) und das Aufnahmeelement (14) mittels Federbolzen (17,18) achsial unverschiebbar miteinander verbunden sind.

7. Adapter nach Anspruch 6, dadurch gekennzeichnet, dass die Federbolzen (18,19) radial bezüglich der Adapterachse im Innern des Hohlkörpers (5) angeordnet sind.

8. Adapter nach Anspruch 6, dadurch gekennzeichnet, dass im Hohlkörper (5) eine Öffnung (20) für das Einresp. Austreiben der Federbolzen (18,19) vorhanden ist.
